# EUROPÄISCHE PATENTSCHRIFT

(11) **EP 3 880 089 B1**
(45) Veröffentlichungstag und Bekanntmachung des Hinweises auf die Patenterteilung: **20.12.2023**
(21) Anmeldenummer: 19831641.6
(22) Anmeldetag: 16.12.2019
(51) Int. Cl.: A61B 17/22

(54) **DRUCKWELLENGERÄT ZUR BEHANDLUNG EINES MENSCHLICHEN ODER TIERISCHEN KÖRPERS**
SHOCK WAVE APPARATUS FOR TREATING A HUMAN OR ANIMAL BODY
APPAREIL À ONDES DE PRESSION DE TRAITEMENT D'UN CORPS HUMAIN OU ANIMAL

(30) Priorität: 21.12.2018 DE 102018133356
(43) Veröffentlichungstag der Anmeldung: 22.09.2021
(73) Patentinhaber: Ferton Holding S.A., 2800 Delémont (CH)
(72) Erfinder: EVANS, Gary, 74200 Allinges (FR); SUMI, Nicolas, 1005 Lausanne (CH)
(74) Vertreter: Müller Schupfner & Partner Patent- und Rechtsanwaltspartnerschaft mbB
(86) Internationale Anmeldenummer: PCT/EP2019/085333
(87) Internationale Veröffentlichungsnummer: WO 2020/127035

(56) Entgegenhaltungen:
- EP-A1- 2 529 792
- EP-A1- 3 135 268
- EP-B1- 2 381 864
- US-A1- 2017 156 973

## Beschreibung

Die vorliegende Erfindung betrifft ein Druckwellengerät zur Behandlung eines menschlichen oder tierischen Körpers mit einem pneumatischen Antrieb zur Erzeugung einer Druckwelle zur Einkopplung in den menschlichen oder tierischen Körper.

Druckwellengeräte zur Behandlung des menschlichen oder tierischen Körpers mit Druckwellen, bei denen das Aufeinanderprallen eines beschleunigten Schlagteils auf einen Prallkörper eine Druckwelle erzeugt, sind an für sich bekannt.

Aus der EP 2 529 792 A1, EP 3 135 268 A1 oder der EP 2 381 864 B1 sind beispielsweise Druckwellengeräte zur Behandlung des menschlichen oder tierischen Körpers bekannt. Die Druckwellengeräte weisen einen pneumatischen Antrieb zur Erzeugung einer Druckwelle auf. Diese wird hier durch ein Schlagteil erzeugt, das pneumatisch beschleunigt wird und gegen ein Übertragungselement schlägt, das die Druckwelle durch Berührung in den Körper einkoppelt. Die Druckwellengeräte weisen daher einen Kompressor als Teil des pneumatischen Antriebs zur Erzeugung von Quellengas auf, wobei der Kompressor einen Kompressormotor aufweist. Die Druckwellengeräte weisen eine Einrichtung zur Einstellung des Druckwellenerzeugungsdrucks z. B mittels der Drehzahl des Kompressormotors auf, d.h. eine Einrichtung zur Einstellung des beschleunigenden Gasdrucks. Bei dem Druckwellengerät der EP 2 381 864 B1 arbeitet der Kompressor kontinuierlich bei unterschiedlichen Leistungen, um in einem Handstück durchstimmbar Druckwellen unterschiedlicher Stärke bereit stellen zu können, was zu einem frühzeitigen Verschleiß des Kompressors führt und mit verschieden lauten Geräuschkulissen verbunden ist.

Es ist bekannt, dass bei einem Druckwellengerät der Kompressor entsprechend des zu erzeugenden Gasdrucks für die Druckwelle angesteuert wird. Hierbei ist in der Regel der Kompressor solange in Betrieb, bis ein vorgegebener maximaler Quellengasdruckwert, der bei einigen Varianten in einem Druckreservoir gespeichert wird, erreicht ist. Nach einem Erreichen des Quellengasdruckwertes wird der Kompressor in der Regel abgeschaltet, um einem Erzeugen eines Überdrucks entgegen zu wirken. Dies hat zur Folge, dass während einer Behandlung der Kompressor immer wieder seinen Betrieb aufnimmt, wodurch eine Hintergrundgeräuschkulisse erzeugt wird, welche mal lauter und mal leiser ist. Dies hat wiederum zur Folge, dass ein Patient dies aktiv wahrnimmt und sich in seinem Wohlbefinden während der Behandlung durch die Hintergrundgeräuschkulisse gestört fühlt. Auch verschleißt der Kompressor relativ schnell.

Bei Druckwellengeräten ist es wünschenswert, dass diese über eine bestimmte Zeitdauer in einem Handstück einen konstanten Druck, insbesondere in Bereichen unterschiedlicher Flussraten, liefern, welcher von einem Nutzer nach Bedarf einstellbar ist. Insbesondere ist es wünschenswert, dass ein konstanter Druck für einen Flussratenbereich, welcher durch eine Schaltfrequenz eines entsprechenden Ventils, wie beispielsweise einem Magnetventil des Handstückes, bestimmt ist, von dem Druckwellengerät bzw. dem pneumatischen Antrieb bereitgestellt wird. Dies ist ein Leistungsbereich oder Lastbereich. Das gilt ebenso für wechselnde Drücke bei fester Schaltfrequenz oder einer Kombination beider Einstellungen. Ferner ist es wünschenswert, dass eine akustische Hintergrundkulisse des Druckwellengerätes während des Betriebes desselben minimiert bzw. konstant ist, wodurch das Wohlbefinden des menschlichen oder tierischen Patienten gesteigert werden kann, da eine Wahrnehmung der Geräuschkulisse bei Monotonie derselben bei dem Patienten in den Hintergrund tritt. Schließlich soll die Lebensdauer des Kompressors verlängert werden.

Angesichts dieser Probleme im Stand der Technik ist es eine Aufgabe der vorliegenden Erfindung, ein Druckwellengerät bereitzustellen, bei dem das Hintergrundgeräusch während des Betriebes des Druckwellengerätes reduziert bzw. konstant wenig wahrnehmbar ist, unabhängig davon, welche Last das Druckwellengerät gerade abruft. Die Last ist im wesentlichen abhängig von der Frequenz des Druckwellengeräts, d.h. mit welcher Schaltfrequenz ein Ventil des Handstückes betrieben wird (Menge an Luft) sowie dem Druck, bei dem das Druckwellengerät betrieben wird (zur Verfügung stehende Geschwindigkeit für das zu beschleunigende Schlagteil). Ferner ist es eine Aufgabe der vorliegenden Erfindung, ein Druckwellengerät bereitzustellen, welches einen regulierten oder regulierbaren, insbesondere veränderlich konstanten Druck liefert.

Diese Aufgaben werden gelöst mit einem Druckwellengerät gemäß Anspruch 1. Weitere Vorteile und Merkmale der vorliegenden Erfindung ergeben sich aus den abhängigen Ansprüchen.

Erfindungsgemäß umfasst das Druckwellengerät zur Behandlung eines menschlichen oder tierischen Körpers einen pneumatischen Antrieb zur Erzeugung einer Druckwelle zur Einkopplung in den menschlichen oder tierischen Körper. Ferner umfasst das Druckwellengerät mindestens einen Kompressor zur Erzeugung von Quellengas (oder "Quellgas") mit mindestens zwei einstellbaren Leistungsniveaus sowie ein Handstück, in welches das Quellengas über eine Verbindungsleitung einleitbar ist und mittels welchem ein Schlagteil beschleunigt werden kann, um die Druckwelle in den menschlichen oder tierischen Körper einzukoppeln. Zudem umfasst das Druckwellengerät eine Druckregulierungsvorrichtung zur Einstellung des Quellengasdrucks auf einen Druckwellenerzeugungsdruck zur Erzeugung der Druckwelle. Der Druckwellenerzeugungsdruck ist der Druck, der unmittelbar auf das Schlagteil wirkt.

Das Druckwellengerät weist ein Handstück auf, in welches das Quellengas einleitbar ist und mittels welchem eine Druckwelle erzeugt wird, sowie eine Druckregulierungsvorrichtung zur Einstellung eines Druckwellenerzeugungsdrucks zur Erzeugung der Druckwelle, wobei mindestens ein Kompressor insbesondere eines Kompressorsystems zur Erzeugung von Quellengas stufenweise auf mindestens zwei Leistungsniveaus einstellbar ist, jedes Leistungsniveau durch einen Bereich an Druckwellenerzeugungsdruckwerten (P_{D_Bi}) und Frequenzen (f_{Bi}) bestimmt ist, und die Druckregulierungsvorrichtung den Druckwellenerzeugungsdruck (P_{D_i}) durch Anpassung des Quellengasdrucks (P_{s_i}) auf jedem Leistungsniveau Lᵢ regelt.

Erfindungsgemäß weist das Druckwellengerät mindestens ein Kompressorsystem auf, welches zur Erzeugung verschiedener Leistungsniveaus stufenweise in verschiedenen Betriebszuständen betreibbar ist. Die Auswahl der unterschiedlichen Leistungsniveaus kann dabei entweder durch eine Voreinstellung abhängig von den eingestellten Betriebsparametern des Druckwellengerätes (Druckwellenerzeugungsdruck- und Frequenz-Bereich) erfolgen oder das Leistungsniveau wird von dem Druckwellengerät selbständig gewechselt, wenn die Druckregulierungsvorrichtung zur Einstellung des Quellengasdrucks auf einen Druckwellenerzeugungsdruck und/oder zur Einstellung der gewünschten der Flussrate, d.h. der Frequenz der Ventilöffnung zur wiederholten Beschleunigung des Schlagteils, nicht mehr in der Lage ist dem Druckwellengerät den erforderlichen Druckwellenerzeugungsdruck und/oder die benötigte Flussrate bereitzustellen.

Mit anderen Worten: Das erfindungsgemäße Druckwellengerät weist ein Kompressorsystem auf, welches zur Erzeugung verschiedener Leistungsniveaus stufenweise in verschiedenen Betriebszuständen betreibbar ist, wobei die Druckregulierungsvorrichtung den Druckwellenerzeugungsdruck durch Anpassung eines ersten Quellengasdrucks innerhalb eines ersten Leistungsniveaus, d.h. innerhalb eines ersten bestimmten Flussratenbereichs, einstellt, insbesondere reduziert, und wobei die Druckregulierungsvorrichtung das Kompressorsystem in einen zweiten Betriebszustand mit einem zweiten Leistungsniveau schaltet, um den Druckwellenerzeugungsdruck einzustellen, insbesondere zu reduzieren, sobald die Druckregulierungsvorrichtung nicht mehr in der Lage ist den erforderlichen Druckwellenerzeugungsdruck und/oder die erforderliche Flussrate bereitzustellen

Der Druckwellenerzeugungsdruck ist der Druck, mit dem das Schlagteil des Druckwellengeräts beschleunigt wird. Die Flussrate ist die Frequenz der Ventilöffnung am Handstück des Druckwellengeräts , d.h. die Anzahl an erzeugten Druckwellen pro Zeiteinheit. Ein bestimmter Leistungsbereich setzt sich aus gewünschtem Druckwellenerzeugungsdruck bei bestimmter Flussrate zusammen. Je höher der gewünschte Druckwellenerzeugungsdruck innerhalb eines bestimmten Leistungsbereichs sein soll, desto niedriger ist die Flussrate und vice versa. Innerhalb eines bestimmten Leistungsbereichs existiert des weiteren ein erster maximaler Druckwellenerzeugungsdruck bei minimaler Flussrate und eine maximale Flussrate bei minimalem Druckwellenerzeugungsdruck. Soll also ein Druckwellenerzeugungsdruck erzeugt werden, der diesen maximalen Druckwellenerzeugungsdruck in dieser bestimmten Leistungsstufe übersteigt oder soll eine Flussrate erzielt werden, die die maximale Flussrate in dieser bestimmten Leistungsstufe übersteigt, so wird von dem Druckwellengerät das Leistungsniveau selbständig gewechselt.

Jedes Leistungsniveau (Lᵢ) wird durch einen Bereich an Druckwellenerzeugungsdruckwerten (P_{D_Bi}) und Frequenzen (f_{Bi}) der Aktivierung der Druckwellen bestimmt. Die Auswahl eines Leistungsniveaus (Lᵢ) erfolgt mit Vorteil anhand einer Tabelle, in der die jeweiligen Bereiche an Druckwellenerzeugungsdruckwerten (P_{D_Bi}) und Frequenzen (f_{Bi}) der Aktivierung der Druckwellen hinterlegt sind.

Die Druckregulierungsvorrichtung ist zur Einstellung unterschiedlicher Druckwellenerzeugungsdrücke P_{D} zur Erzeugung der Druckwelle ausgebildet,. Hierzu ist beispielsweise unmittelbar vor oder an einem Einlass des Handstücks die Druckregulierungsvorrichtung zum Anpassen des Quellengasdrucks, insbesondere zum Bereitstellen eines im Wesentlichen konstanten, vorbestimmten Druckwellenerzeugungsdruck P_{D}, in dem Handstück angeordnet. Mit anderen Worten: Während der Kompressor und/oder die Kompressoren in einem diskret ansteuerbaren Betriebszustand ein bestimmtes Leistungsniveau bei konstanter Drehzahl abdecken, in dem ein aktueller Quellengasdruck beispielsweise betriebsbedingten bzw. lastabhängigen Schwankungen unterliegt, ist es vorgesehen, dass eine Einstellung des Druckwellenerzeugungsdrucks mittels der Druckregulierungsvorrichtung bei konstanter Drehzahl erfolgt. Vorzugsweise ist das Kompressorsystem derart ausgelegt, dass die diskret einstellbaren Leistungsniveaus nicht überlappen. Das Kompressorsystem kann beispielsweise aus einem einzelnen oder mehreren Kompressoren bestehen.

Es ist ferner denkbar, die Druckregulierungsvorrichtung in den pneumatischen Antrieb oder an einer beliebigen Position in der Verbindungsleitung anzuordnen. Vorliegend ist unter dem Begriff "im Wesentlichen konstanten Druckwellenerzeugungsdruck" ein Gasdruck zu verstehen, welcher höchstens um ± 0 bis 0,5 bar von dem vorbestimmten Druckwellenerzeugungsdruck P_{D}, also dem vorgegebenen Sollwert des Gasdrucks für die zu erzeugende Druckwelle, abweicht. Vorzugsweise ist die Druckregulierungsvorrichtung dazu ausgelegt, dass die Abweichungen von dem gewünschten Druckwellenerzeugungsdruck, d. h. eine Einstellgenauigkeit des Druckwellenerzeugungsdrucks, stets kleiner sind als 0,2 bar, bevorzugt kleiner als 0,15 bar und besonders bevorzugt kleiner als 0,1 bar. Dadurch läßt sich eine für eine betriebssichere Nutzung des Druckwellengeräts erforderliche Einstellgenauigkeit realisieren.

Gleiches gilt sinngemäß für die entsprechenden Flussraten, die bei bestimmten Druckwellenerzeugungsdruckwerten zur Verfügung stehen müssen.

Vorzugsweise umfasst das Kompressorsystem mehrere Kompressoren, zwischen denen, beispielsweise zum Einstellen eines gewünschten Leistungsniveaus, gewechselt wird. Mit anderen Worten: der einzelne Kompressor wird nicht in einem bestimmten Betriebszustand betrieben, um den Quellengasdruckbereich anzusteuern bzw. festzulegen, sondern es wird zwischen den Kompressoren gewechselt (oder diese werden einzeln zugeschaltet), die jeweils für die Erzeugung eines einzelnen Betriebszustands mit einem entsprechenden Leistungsniveau verantwortlich sind. Das jeweilige Leistungsniveau definiert sich gemäß der Erfindung aus den Betriebsparametern Druck und Frequenz (Flussrate) des Druckwellengeräts, die den Verbrauch (Luftmenge pro Zeiteinheit) an pneumatischen Mitteln des Druckwellengerätes bestimmen..

Durch die Druckregulierungsvorrichtung kann ein vorgegebener Druckwellenerzeugungsdruck P_{D} eingestellt werden, beispielsweise bevor der Gasdruck in das Handstück geleitet wird, um dort ein Schlagteil zu beschleunigen und eine Druckwelle zu erzeugen. Dies hat den Vorteil, dass ein Druckwellenerzeugungsdruckbereich für ein bestimmtes Leistungsniveau, z.B. einen bestimmten Flussratenbereich, bereitgestellt werden kann, und zwar unabhängig von einer konkret genutzten Schaltfrequenz eines Ventiles, wie beispielsweise einem Magnetventil, im Handstück also unabhängig von der Aktivierungsdauer des Druckwellenerzeugungsdrucks und/oder unabhängig von einer Kompressorleistung eines Kompressors bzw. einen aktuell veranlassten Quellengasdruck durch den oder die Kompressoren.

Ferner können ein oder mehrere entsprechende Kompressoren zur Erzeugung desQuellengasdrucks in einem bestimmten Leistungsbereich, bevorzugt während einer Behandlung, durchgängig betrieben werden, so dass eine monotone Hintergrundgeräuschkulisse hörbar ist. Ferner kann der Betriebszustand der Kompressoren derart ausgewählt sein, dass sich eine besonders vorteilhafte Geräuschkulisse einstellt. Insbesondere lassen sich nur solche Drehzahlen auswählen, bei denen starke Vibrationen oder Resonanzen nicht auftreten. Im Falle diskret einstellbarer Drehzahlen läßt sich die Vibration der Druckerzeugungseinrichtung gezielt durch konstruktive Maßnahmen unterdrücken. Dabei wird die Drehzahl des oder der Kompressoren im Wesentlichen durch dessen Spannung bzw. Betriebsspannung festgelegt, die diskret einstellbar ist. Ebenso ist es denkbar, dass eine elektrische Steuerung die Drehzahl des Kompressors auf einen von mehreren fest vorwählbaren Werten fixiert. Die diskreten Leistungsniveaus des oder der Kompressoren sind dabei festen Drehzahlen des oder der Kompressoren zugeordnet. Hierdurch kann insbesondere eine subjektive Wahrnehmbarkeit der Hintergrundgeräuschkulisse während des Betriebes des Druckwellengerätes reduziert werden, wodurch wiederum das Wohlbefinden eines Patienten während der Behandlung gesteigert werden kann. Durch die konstante Drehzahl wird dieser weniger belastet als beim wiederholten Ein-/Ausschalten bzw. als bei wiederholten Drehzahländerungen. Mit anderen Worten: Der oder die Kompressoren arbeiten bei fester Spannung mit fester Drehzahl, die Lastunterschiede (Druck und Flussrate) bewirken lediglich eine unterschiedlich hohe Stromaufnahme der Motoren.

Bevorzugt weist die Druckregulierungsvorrichtung ein Ablassventil auf, das insbesondere ein Ventil, insbesondere ein Magnetventil, aufweist. Beispielsweise umfasst die Druckregulierungsvorrichtung ein Proportionalventil. Ein Proportionalventil läßt beispielsweise stetige bzw. kontinuierliche Übergänge zwischen einem vollständig geöffneten und einem vollständig geschlossenen Zustand zu. Dabei kann es sich beispielsweise um ein elektromagnetisch gesteuertes oder ein mediumgesteuertes Proportionalventil handeln.

Die Druckregulierungsvorrichtung wird in vorteilhafter Weise dazu eingesetzt, um an bzw. in dem Handstück einen konstanten Druck, nämlich den Druckwellenerzeugungsdruck P_{D} zur Erzeugung der Druckwelle, bereitzustellen, so dass gleichmäßige Druckwellen über das Handstück in den zu behandelnden Körper einleitbar sind.

Allgemein versteht man unter einem Druckminderer ein Druckventil zum Einbau in ein Schlauch- oder ein Leitungssystem, wobei das Druckventil trotz unterschiedlicher Drücke auf einer Eingangsseite (Eingangsdruck), vorliegend dem Quellengasdrucks Pₛ entsprechend, dafür sorgt, dass auf der Ausgangsseite, welche vorliegend in das Handstück führt, ein bestimmter Ausgangsdruck, vorliegend dem Druckwellenerzeugungsdruck P_{D} entsprechend, nicht überschritten wird. Durch das Vorsehen einer Druckregulierungsvorrichtung wird der Quellengasdruck Pₛ auf den benötigten Druckwellenerzeugungsdruck P_{D} zur Erzeugung der Druckwelle gewandelt. Ein Druckminderer hat somit den Vorteil, dass gleichmäßige Druckwellen in dem Druckwellengerät erzeugt werden können.

Gemäß der vorliegenden Erfindung weist die Druckregulierungsvorrichtung mindestens einen Drucksensor zum Bestimmen eines Druckwellenerzeugungsdrucks P_{D} auf. Hierdurch kann in einfacher Weise überprüft werden, ob die Druckregulierungsvorrichtung eine entsprechende Regulierung, insbesondere eine Druckminderung, vorgenommen hat. Vorzugsweise ist die Druckregulierungsvorrichtung als Regelkreis ausgelegt. Es ist denkbar, einen weiteren Drucksensor vorzusehen. Durch Anordnung eines weiteren Drucksensors kann der Gasdruck, d.h. der Quellengasdruck, insbesondere vor Eintritt in das Handstück, zu Überprüfung erfasst werden. Abhängig von dem erfassten Quellengasdruck und/oder abhängig von dem erfassten Druckwellenerzeugungsdruck P_{D} - je nachdem wo der mindestens eine Drucksensor angeordnet ist - kann ein, insbesondere in einer Steuerung hinterlegter, Druckwellenerzeugungsdruck P_{D} eingestellt werden.

Insbesondere ist es vorgesehen, dass das Kompressorsystem derart angesteuert bzw. konfiguriert ist, dass das diskrete Leistungsniveau stets einen Quellengasdruck bereitstellt, der auf den gewünschten Druckwellenerzeugungsdruck reduziert werden kann, um sicherzustellen, dass dieser am Handstück zuverlässig und unabhängig von der Schaltfrequenz des Druckwellenerzeugungsdrucks bereitgestellt werden kann. Mit anderen Worten: es wird vermieden, dass der bereitgestellte Quellengasdruck nicht ausreicht, um den gewünschten Druckwellenerzeugungsdruck zu allen Zeiten, d.h. innerhalb eines Flussratenbereichs, zu realisieren.

Gemäß einer Ausführungsform des Druckwellengerätes ist in einer Steuerung zu einem Leistungsniveau Lᵢ mit i≥1 ein vorbestimmter Bereich an Druckwellenerzeugungsdruckwerten P_{D_i} und ein vorbestimmter Bereich an Schaltfrequenzen fᵢ mit i≥1 hinterlegt, also eine Drucktabelle, wobei die Druckregulierungsvorrichtung dazu ausgebildet ist, in Abhängigkeit des gewünschten Druckwellenerzeugungsdrucks P_{D_i}, z.B. für einen gewünschten Bereich an Schaltfrequenzen fᵢ das notwendige Leistungsniveau Lᵢ des Kompressorsystems einzustellen.

Wie hierin vorgeschlagen, wird mittels eines oder mehrerer Kompressoren zunächst ein innerhalb eines bestimmten Leistungsniveaus liegender erster Quellengasdruck P_{s_i} mit i≥1 erzeugt, welcher insbesondere anschließend von einem Drucksensor erfasst werden kann. Der erfasste Quellengasdruck P_{s_i} kann mit vorgegebenen bzw. gewünschten Druckwellenerzeugungsdrücken P_{D_i}, welche in einer Steuerung hinterlegt sind, abgeglichen bzw. verglichen werden. Jedem Quellengasdruckwert P_{s_i} ist dabei, insbesondere bei einer vorgegebenen Frequenz des Druckwellengeräts, d.h. zeitabhängige Anzahl der Hübe des Schlagteils, zur Erzeugung der Druckwellen, ein Bereich an Druckwellenerzeugungsdrücken P_{D_i} zugeordnet. Eine solche Zuordnung erfolgt insbesondere in Abhängigkeit einer eingestellten Frequenz an dem Druckwellengerät. Der Druckwellenerzeugungsdruck P_{D_i} ist somit variabel, wobei variabel vorliegend so zu verstehen ist, dass anhand eines bestimmten vorgegebenen, insbesondere eingestellten, Quellengasdruckbereichs bzw. Lastbereichs, der jeweils einen maximalen Quellengasdruckwert P_{s_i}, hat, ein gewünschter Druckwellenerzeugungsdruck P_{D_i} eingestellt werden kann, welcher einem bestimmten Bereich an vorgegebenen, gewünschten Druckwellenerzeugungsdrücken P_{D_i} zugeordnet ist, und ein anderer bestimmter Quellengasdruckbereich bzw. Lastbereich, der jeweils einen anderen maximalen Quellengasdruckwert P_{s_i±1}, hat, einem anderen Bereich an Druckwellenerzeugungsdrücken P_{D_i±1} zugeordnet ist.

Abhängig von den bestimmten, stufenweise vorgegebenen Lastbereichen bzw. Leistungsniveaus Lᵢ wird mittels der Druckregulierungsvorrichtung, bei bestimmten Flussraten, der gewünschte Druckwellenerzeugungsdruck P_{D_i} oder ein anderer gewünschter Druckwellenerzeugungsdruck P_{D_i±1} auf einem anderen Leistungsniveau L_{i±1} eingestellt.. Dies hat den Vorteil, dass gleichmäßige Druckwellen mit einem gewünschten Druckwellenerzeugungsdruck P_{D_i} während einer Behandlung erzeugbar sind, und zwar mittels stufenweise angesteuerter, diskreter Leistungsniveaus Lᵢ. Der bzw. die Kompressoren arbeitet somit in diskreten Stufen.

Bevorzugt weist das Druckwellengerät mindestens zwei Kompressoren auf. Jeder der mindesten zwei Kompressoren kann dabei mit einem gleichen oder einem unterschiedlichen Drehzahllevel betrieben werden, insbesondere bei verschiedenen Leistungsniveaus. Hierdurch können die Kompressoren auf verschiedenen, insbesondere diskreten, Leistungsniveaus betrieben werden. Vorliegend kann ein diskretes Leistungsniveau bei einem vorgegebenen Quellengasdruck und einer vorgegebenen Flussrate realisiert sein. Durch die mindestens zwei Kompressoren wird insbesondere erreicht, dass ein Rauschen, also eine Hintergrundgeräuschkulisse, des Druckwellengerätes reduziert wird, insbesondere wenn das Druckwellengerät in einem Bereich einer geringen Flussrate und/oder bei einem geringen Quellengasdruck genutzt wird. Denn zur Erzeugung eines bestimmten Quellengasdrucks P_{s_i} können beispielsweise zwei oder mehrere Kompressoren eingesetzt werden, welche jeweils bei einer geringeren Leistung betrieben werden.

Bevorzugt sind die mindestens zwei Kompressoren mit einem oder mit je einem Kompressormotor zur Ansteuerung der Kompressoren verbunden. Der Kompressor bzw. jeder Kompressor kann somit durch den entsprechenden Motor bei einer vorgegebenen Leistung oder einer vorgegebenen Frequenz angesteuert werden, wodurch ein vorbestimmtes Leistungsniveau an dem entsprechenden Kompressor einstellbar ist. Hierdurch kann vorteilhafterweise ein stetig wechselnder Lärm des Druckwellengerätes reduziert werden und der Verschleiß reduziert sich durch einen konstanten Betrieb.

Gemäß einer bevorzugten Ausführungsform des Druckwellengerätes ist, insbesondere zwischen dem mindestens einen Kompressor und der Druckregulierungsvorrichtung, eine Kühlfalle zum Auffangen von abgekühltem, kondensiertem Quellengas angeordnet. Durch die Kühlfalle kann folglich kondensiertes Gas, insbesondere kondensierte Luft, aufgefangen werden. Hierdurch wird verhindert, dass kondensiertes Gas, d. h. eine Flüssigkeit, in das Handstück geleitet wird. Hierdurch wird lediglich Quellengas in das Handstück geleitet. Somit kann langfristig vermieden werden, dass sich in dem Handstück Feuchtigkeit absetzt und somit beispielsweise zur Schimmelbildung, Korrosion oder dergleichen führt.

Weiter bevorzugt ist, insbesondere zwischen dem Kompressor oder den Kompressoren und der Druckregulierungsvorrichtung, ein Überdruckventil zum Ableiten eines Überdrucks des Quellengases vorgesehen. Hierdurch kann vermieden werden, dass das Druckwellengerät durch einen erzeugten Überdruck beschädigt oder gar zerstört wird. Vor allem bei einem Gasdruck größer als 5 bar, bevorzugt bei einem Druck bis zu 25 bar, kommt das Überdruckventil zum Einsatz.

Bevorzugt ist jeder Kompressor dazu ausgebildet, stufenweise verschiedene, insbesondere diskrete, Volumenströme zu erzeugen, um verschiedene Quellengasdrücke und/oder Flussraten des Quellengases zu generieren. Jeder Kompressor kann folglich hinsichtlich seiner Kompressorleistung eingestellt bzw. geregelt werden, so dass jeder Kompressor stufenweise verschiedene Volumenströme erzeugen kann und damit verschiedene Quellengasdrücke und/oder Flussraten des Quellengases generieren kann. Die Kompressorleistung dient somit zur Erzeugung eines Quellengases bei einer gestuften, d.h. diskreten Drehzahl des Kompressormotors, Bevorzugt weist jeder Kompressor einen eigenen Kompressormotor auf. Insbesondere weist jeder Kompressor einen Gleichstrommotor (DC-Motor) auf. Hierdurch kann jeder Kompressor bei fester Drehzahl (also fester vorgegebener Spannung) aber unterschiedlicher Stromaufnahme bei unterschiedlichen Druckwerten und/oder unterschiedlichen Flussraten innerhalb eines Leistungsniveaus betrieben werden. Dies hat den Vorteil, dass ein Hintergrundrauschen des Druckwellengerätes insgesamt reduziert werden kann.

Gemäß einer bevorzugten Ausführungsform des Druckwellengerätes ist das Druckwellengerät derart konfiguriert, dass das Druckwellengerät nach einem, insbesondere jedem, Gebrauch des Druckwellengeräts mittels eines ansteuerbaren Entlüftungsventils, insbesondere eines Magnetventils, entlüftbar ist. Über das Entlüftungsventil kann in vorteilhafter Weise ein in einem Kreislauf des Druckwellengerätes zurückgebliebenes Quellengas aus dem Kreislauf entfernt werden. Das Entlüftungsventil arbeitet bevorzugt autark. Es ist jedoch denkbar, dass das Entlüftungsventil durch eine Steuerung zusätzlich angesteuert werden kann. Insbesondere kann durch ein solches Entlüftungsventil vermieden werden, dass sich Quellengas in dem Kreislauf bzw. dem Druckwellengerät langfristig absetzt und ggf. kondensiert. Hierdurch kann in einfacher Weiser dem Wachstum von Bakterien oder dergleichen entgegengewirkt werden.

Ein weiterer Aspekt, der nicht beansprucht wird, betrifft ein Verfahren zur Behandlung eines menschlichen oder tierischen Körpers mit Druckwellen, insbesondere erzeugt mit einem hierin beschriebenen Druckwellengerät, wobei das Verfahren folgende Schritte umfasst:
- Einschalten des mindestens einen Kompressors zur Erzeugung von Quellengas,
- Durchleiten des Quellengases von dem Kompressor zu dem Handstück, wobei der mindestens eine Kompressor in einen diskret ansteuerbaren Betriebszustand, dem ein diskretes Leistungsniveau L zugeordnet ist, eingestellt wird,
- wobei der Druckwellenerzeugungsdruck durch Anpassung eines ersten Quellengasdrucks innerhalb des Leistungsniveaus L durch eine Druckregulierungsvorrichtung bei einer bestimmten Flussrate geregelt wird,
- und wobei der Druckwellenerzeugungsdruck durch Erhöhung oder durch Minderung des Leistungsniveaus L geregelt wird.

Mit Einschalten des Kompressors oder der Kompressoren kann ein erster Druck des Quellengases P_{s_i} mit i≥1 innerhalb eines diskreten Leistungsniveaus L eingestellt werden. Die möglichen vorgegebenen bzw. gewünschten Druckwellenerzeugungsdrücke P_{D_i} mit i≥1, welche jeweils einem bestimmten diskreten Leistungsniveau Lᵢ bei einer bestimmten Flussrate bzw. Frequenz fᵢ zugeordnet sind, können insbesondere in einer Steuerung hinterlegt sein und einem Nutzer beim Einschalten des Druckwellengerätes zum individuellen Auswählen angezeigt werden. Es ist ferner möglich, dass ein Nutzer einen vorgegebenen Druckwellenerzeugungsdruck P_{D_i} manuell eingibt. Hierbei kann dann zunächst ein erster Quellengasdruck P_{s_i} bei einem ersten Leistungsniveau Lᵢ erzeugt werden, welcher mittels der Druckregulierungsvorrichtung auf einen ersten Druckwellenerzeugungsdruck P_{D_i} reduziert werden kann Der eingestellte Druckwellenerzeugungsdruck P_{D_i} kann dann zu einem Einlass des Handstückes strömen.

Während das Quellengas zu dem Handstück strömt, wird, insbesondere von einem Drucksensor, der Gasdruck erfasst. Abhängig davon, wo der Drucksensor angeordnet ist, erfasst dieser entweder den Quellengasdruck (vor Passage der Druckregulierungsvorrichtung) oder den Druckwellenerzeugungsdruck (nach Passage der Druckregulierungsvorrichtung) oder es werden mit zwei Sensoren beide Drücke gemessen. Je nach Anordnung des mindestens einen Drucksensors wird folglich der Quellengasdruck P_{s_i} oder der Druckwellenerzeugungsdruck P_{D_i} erfasst. Die Druckregulierungsvorrichtung wird je nach erfasstem Quellengasdruck P_{s_i} des Quellengases bzw. des Druckwellenerzeugungsdrucks P_{D_i} angesteuert. Hierdurch wird folglich erreicht, dass das Handstück einerseits stets mit einem konstanten Druckwellenerzeugungsdruck P_{D_i} versorgt wird, so dass gleichmäßige Druckwellen an einem Ende des Handstückes zu Übertragung in den menschlichen oder tierischen Körper erzeugt werden können, andererseits der oder die Kompressoren nicht stets unter Volllasst sondern nur in einem Bereich, d.h. auf dem diskretenLeistungsniveau Lᵢ konstant arbeiten, in den der benötigte Druckwellenerzeugungsdruck P_{D_i} fällt.

Bevorzugt umfasst das Verfahren ferner die Schritte:
- Schließen eines Entlüftungsventils, und/oder
- ein zumindest teilweises Öffnen oder ein zumindest teilweises Schließen einer Druckregulierungsvorrichtung bzw. ein Regeln der Druckregulierungsvorrichtung zum Durchleiten des Quellengases von einem Kompressor zu einem Handstück.

Bei einem Schritt wird das Entlüftungsventil geschlossen, um zu vermeiden, dass ein, insbesondere in dem mindestens einen Kompressor, erzeugtes Quellengas, welches zur Erzeugung der Druckwelle vorgesehen ist, also der Quellengasdruck P_{s_i} mit i≥1, aus dem Druckwellengerät entweicht. Ferner kann die Druckregulierungsvorrichtung wenigstens teilweise geöffnet oder teilweise geschlossen werden, so dass ein erzeugtes Quellengas, nach einer Druckminderung oder einer Druckerhöhung, und damit ein Druckwellenerzeugungsdruck P_{D_i}, zu dem Handstück strömen kann.

Bevorzugt wird nach einem Erfassen des tatsächlich erzeugten Druckwellenerzeugungsdrucks geprüft, ob der erfasste Druckwellenerzeugungsdruck einem gewünschten bzw. vorbestimmten Druckwellenerzeugungsdruck entspricht und in Abhängigkeit einer Differenz zwischen erfasstem Druckwellenerzeugungsdruck und gewünschtem Druckwellenerzeugungsdruck
a) wird der Zustand der Druckregulierungsvorrichtung unverändert beibehalten, sofern die Differenz etwa Null ist;
b) wird die Druckregulierungsvorrichtung zumindest teilweise geöffnet bzw. wird der Quellengasdruck P_{s_i} reduziert, sofern die Differenz positiv ist, d.h. der erfasste Druckwellenerzeugungsdruck über dem gewünschten Druckwellenerzeugungsdruck liegt, und
c) wird die Druckregulierungsvorrichtung zumindest teilweise geschlossen bzw. wird der Quellengasdruck P_{s_i} erhöht, sofern die Differenz negativ ist, d.h. der erfasste Druckwellenerzeugungsdruck unter dem gewünschten Druckwellenerzeugungsdruck liegt.

Vorliegend wird der Quellengasdruck P_{s_i} erhöht oder erniedrigt oder beibehalten, je nachdem was eine Auswertung der Differenz zwischen dem erfassten Druckwellenerzeugungsdruck und dem gewünschten Druckwellenerzeugungsdruck ergibt. Der erfasste Druckwellenerzeugungsdruck P_{D_i} resultiert aus dem an dem mindestens einen Kompressor erzeugten Quellengasdruck P_{s_i}. Jeder Druckwellenerzeugungsdruck P_{D_i} ist dabei einem Leistungsniveau Lᵢ, also einem konstanten Drehzahlberich des oder der Kompressoren, zugeordnet.

Wird bei einem Erfassen des Druckwellenerzeugungsdrucks P_{D_i} festgestellt, dass dieser über einem gewünschten, d.h. einem vorgegebenen Druckwellenerzeugungsdruck P_{D_i} liegt, so wird die Druckregulierungsvorrichtung zumindest teilweise geöffnet oder weiter geöffnet, um Quellengas abzulassen, wodurch der Druckwellenerzeugungsdruck P_{D_i} reduziert wird. Wird ein Druckwellenerzeugungsdruck P_{D_i} unterhalb des gewünschten Druckwellenerzeugungsdrucks P_{D_i} erfasst, so wird die Druckregulierungsvorrichtung zumindest teilweise geschlossen oder weiter geschlossen, um einen höheren Druckwellenerzeugungsdruck P_{D_i} zu erzeugen. Wird hingegen ein Druckwellenerzeugungsdruck P_{D_i} im Wesentlichen gleich dem gewünschten Druckwellenerzeugungsdruck P_{D_i} erfasst, so wird der Zustand der Druckregulierungsvorrichtung unverändert beibehalten.

Durch Öffnen oder Schließen der Druckregulierungsvorrichtung, insbesondere des Proportionalventils, allein kann folglich der gewünschte Druckwellenerzeugungsdrucks P_{D_i}, eingestellt werden, wobei ein Öffnen oder Schließen der Druckregulierungsvorrichtung nicht voraussetzt, dass der von dem mindestens einen Kompressor erzeugte Quellengasdruck P_{s_i} geändert werden muss. Der Quellengasdruck P_{s_i} kann bei Bedarf jedoch geändert werden, insbesondere durch Einstellen des mindestens einen Kompressors auf einen anderen Leistungslevel. Der "Bedarf" ergibt sich dann, wenn die Differenz so groß ist, dass der gewünschte Druckwellenerzeugungsdruck P_{D_i} nur erreicht werden kann, wenn eine andere (höhere oder niedrigere) Stufe des Quellengasdrucks P_{s_i} eingestellt wird. Gleiches gilt sinngemäß bei Änderungen der Flussraten.

Vorliegend ist unter dem Begriff "im Wesentlichen gleich" bzw. unter dem Begriff eine "Differenz etwa Null "ein Druckwellenerzeugungsdruck P_{D_i} zu verstehen, welcher höchstens um ± 0 bis 0,5 bar von dem vorgegebenen Druckwellenerzeugungsdruck P_{D_i}, also dem vorgegebenen Sollwert des Druckwellenerzeugungsdrucks P_{D_i}, abweicht. In der Steuerung kann beispielsweise zu einem Quellengasdruck P_{s_i} ein Bereich an einstellbaren Druckwellenerzeugungsdrücken P_{D_i}, insbesondere in Abhängigkeit einer Frequenz, hinterlegt sein. Hierdurch wird in einfacher Weise ein Stufenmodell für den zu erzeugenden Quellengasdruck P_{s_i} realisiert.

Ein "Öffnen" oder "Schließen" der Druckregulierungsvorrichtung ist vorliegend nur beispielhaft erläutert. Es ist denkbar, dass die Druckregulierungsvorrichtung gerade in umgekehrter Richtung arbeitet, so dass ein Öffnen oder Schließen der Druckregulierungsvorrichtung gerade das Gegenteil, wie hierin beschrieben, bewirkt. Unter "Öffnen"/"Schließen" fallen auch andere Regelungen zur Einstellung eines Drucks.

Bevorzugt wird das mindestens eine Leistungsniveau stufenweise erhöht oder erniedrigt, wenn für den auf einer bestimmten Leistungsstufe Lᵢ₋₁ maximalen Quellengasdruck P_{s_(i-1)} ≤ P_{D_i} gilt oder für den erforderlichen bzw. benötigten Druckwellenerzeugungsdruck P_{D_i} ≤ P_{s_(i-1)} gilt, wobei für ein Erhöhen oder ein Erniedrigen des Leistungsniveaus die Anpassung der Kompressordrehzahl(en) und/oder die Hinzunahme weiterer Kompressoren oder das Abschalten einzelner Kompressoren erfolgt. Es ist denkbar, eine Zuordnung eines Bereiches an möglichen Druckwellenerzeugungsdrücken P_{D} bei einer bestimmten Flussrate bzw. Frequenz f oder für einen bestimmten Frequenzbereich in einer Steuerung zu hinterlegen, um das Leistungsniveau L zu definieren. Ferner kann in der Steuerung eine einzustellende Spannung U hinterlegt sein, mit welcher der mindestens eine Kompressor betrieben werden muss, um den erwünschten Quellengasdruck Pₛ bei einem eingestellten Leistungsniveau zu erreichen, um den gewünschten Druckwellenerzeugungsdruck P_{D} bei gewünschter Frequenz zu erreichen. Insbesondere läßt sich für ein durch den Anwender ausgewähltes Paar aus Druckwellenerzeugungsdruck und Frequenz eine Spannung am Kompressor auswählen. Beispielsweise ist es vorstellbar, dass eine Kontrolleinrichtung anhand des gewünschten Druckwellenerzeugungsdrucks und der eingestellten Frequenz, die vom Anwender an einer Mensch-Maschinen-Schnittstelle eingegeben werden, automatisch die Spannung am Kompressor einstellt, die einen Leistungsbereich veranlasst, der wiederum den gewünschten Druckwellenerzeugungsdruck umfasst. Dadurch ist es in vorteilhafter Weise möglich, einen Betriebszustand für den Kompressor auszuwählen, der besonders vorteilhaft ist für einen geringen Verschleiß und eine geringe Geräuschkulisse, da der oder die Kompressoren bei fester Spannung stets mit konstanter Drehzahl innerhalb einer Leistungsstufe L betrieben werden. Insbesondere ist es auch vorstellbar, dass der Kompressor für die diskret ansteuerbaren Spannungen entsprechend optimiert bzw. ausgelegt wird, um einen vergleichsweise leisen Betrieb und eine hohe Lebensdauer sicherzustellen.

Weiter bevorzugt ist die Einstellung des vorgegebenen Druckwellenerzeugungsdrucks bei dem vorgeschlagenen Verfahren, insbesondere während einer Behandlung, veränderbar. Wird beispielsweise während einer Behandlung der vorgegebene Druckwellenerzeugungsdruck verändert, so wird die Druckregulierungsvorrichtung bei Überschreiten oder Unterschreiten eines dem geänderten Druck angepassten Druckwellenerzeugungsdrucks weiter geöffnet bzw. weiter geschlossen. Die Druckregulierungsvorrichtung wird also in Abhängigkeit von dem erfassten Druck des Quellengases dann angesteuert, wenn der erfasste Druckwellenerzeugungsdruck nicht mehr mit dem vorgegebenen oder dem geänderten vorgegebenen Druckwellenerzeugungsdruck übereinstimmt, insbesondere nicht mehr im Rahmen vordefinierter Fehlerschranken des Druckwellenerzeugungsdrucks, übereinstimmt.

Weiter bevorzugt erfolgt ein Öffnen und/oder Schließen der Druckregulierungsvorrichtung auch in Abhängigkeit einer Frequenz, mit welcher das Druckwellengerät betrieben wird. Abhängig von der Flussrate, d.h. wie oft innerhalb einer Zeiteinheit das Schlagteil des Druckwellengeräts bei vorgegebenen Druckwellenerzeugungsdruck beschleunigt wird, wird mehr oder weniger Luft verbraucht, die durch die Druckregulierungsvorrichtung geregelt wird. Erst wenn die Druckregulierungsvorrichtung bei vorgegebenem Druckwellenerzeugungsdruck eine benötigte Flussrate bzw. Frequenz nicht mehr erreicht bzw. diese auch bei einer niedrigeren Leistungsstufe erreicht werden kann, wird der oder die Kompressoren auf eine höhere oder niedrigere Leistungsstufe gesteuert.

Weiter bevorzugt ist in einer Steuerung zu einem Bereich von Druckwellenerzeugungsdrücken P_{D_i} und einem Bereich an Frequenzen fᵢ ein bestimmtes Leistungsniveau Lᵢ hinterlegt, dem eine feste Spannung zugeordnet ist, die eine bestimmte Drehzahl am Kompressor bzw. an den Kompressoren auslöst oder sukzessive Kompressoren zu- oder abschaltet, um ein Verfahren, wie hierin beschrieben, durchzuführen. Die Steuerung kann ferner dazu ausgebildet sein, die verschiedenen Ventile, insbesondere unter anderem das Entlüftungsventil, anzusteuern. In der Steuerung sind bevorzugt auch diskrete Werte des Quellengasdrucks P_{s_i} hinterlegt, welche zu einem vorgegebenen Druckwellenerzeugungsdruck P_{D_i} eine Spannung und/oder eine Frequenz zum Ansteuern der Kompressoren, und/oder eine Frequenz, beispielsweise bei welcher die Ventile in dem Handstück schalten oder der mindestens eine Kompressor betrieben werden soll, vorgegeben.

Weiter bevorzugt weist der mindestens eine Kompressor des Druckwellengerätes zum Ausführen des Verfahrens eine Mehrzahl an einstellbaren, stufenweisen Leistungsniveaus auf, mit welchem der mindestens eine Kompressor betrieben werden kann, um einen Quellengasdruck P_{s_i} zu erzeugen. Das jeweilige Leistungsniveau läßt sich insbesondere durch die Festlegung einer Spannung einstellen, die dem gewünschten Druckwellenerzeugungsddruck und der eingestellten Frequenz zugeordnet ist. Es ist denkbar, dass der mindestens eine Kompressor bei verschiedenen Leistungsniveaus verschiedene Quellengasdrücke erzeugt, wie beispielsweise bei sechs verschiedenen Leistungsniveaus einen Quellengasdruck P_{s_i} von 2 bar, 5 bar, 8 bar oder 10 bar. Mittels der Druckregulierungsvorrichtung kann dann ein Druckwellenerzeugungsdruck P_{D_i} von 0-2 bar, 2-5 bar, 5-8 bar oder 8-10 bar bei bestimmten Frequenzen erzeugt werden. Eine Zuordnung der verschiedenen Leistungsniveaus, mit den maximal möglichen Quellengasdrücken P_{s_i}, zu dem entsprechenden gewünschten Druckwellenerzeugungsdruck P_{D_i} bei bestimmtzer Frequenz ist beispielhaft nachfolgender Tabelle zu entnehmen, welche insbesondere eine Minderung des Quellengasdruckes auf den gewünschten Druckwellenerzeugungsdruck wiedergibt:

| | | | | | | |
|---|---|---|---|---|---|---|
| Leistungsniveau | 1 | 2 | 3 | 4 | 5 | 6 |
| Maximaler Quellengasdruck P_{s_i} [bar] | 2 | 5 | 5 | 8 | 8 | 10 |
| Maximaler Druckwellenerzeugungsdruck P_{D_i} [bar] | 0-2 | 2-5 | 2-5 | 5-8 | 5-8 | 8-10 |
| Frequenz f [Hz] | 1-10 | 1-10 | 10-60 | 1-10 | 10-60 | 1-60 |

Weitere Vorteile und Merkmale der vorliegenden Erfindung ergeben sich aus der nachfolgenden Beschreibung bevorzugter Ausführungsformen mit Bezug auf die beigefügten Figuren. Es versteht sich, dass einzelne in den jeweiligen Figuren gezeigte Ausführungsformen Merkmale aufweisen können, die auch in anderen Ausführungsformen zum Einsatz gelangen können, auch wenn dies nicht explizit genannt ist, und sofern dies nicht aufgrund technischer Gegebenheiten oder explizit ausgeschlossen wurde. Es zeigen:
- Figur 1: ein Druckwellengerät gemäß der vorliegenden Erfindung,
- Figur 2: ein Schaltbild des Druckwellengeräts gemäß Figur 1,
- Figur 3: eine Alternative eines Schaltbildes des Druckwellengeräts gemäß Figur 1,
- Figur 4: ein Ablaufschema eines vorgeschlagenen Verfahrens zur Behandlung eines menschlichen oder tierischen Körpers mit Druckwellen, und
- Figur 5: eine Visualisierung des hierin vorgeschlagenen Stufenmodells, welches einen Zusammenhang zwischen einem Druckwellenerzeugungsdruck P_{D_i} und einer Frequenz, mit welcher der mindestens eine Kompressor betrieben wird, wiedergibt.

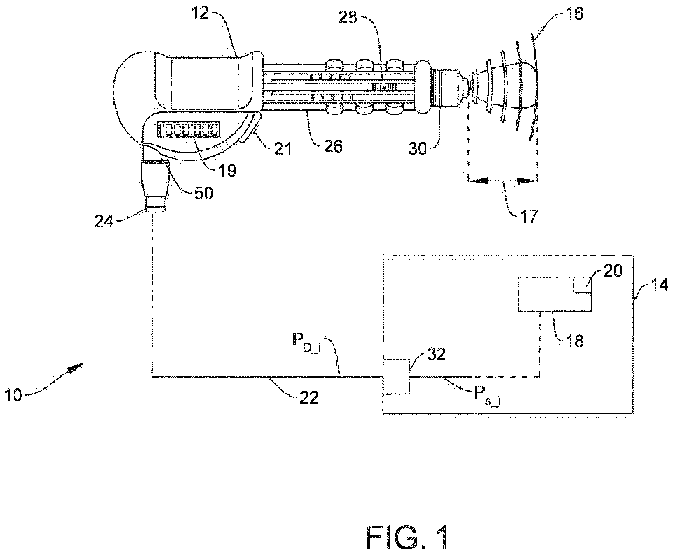

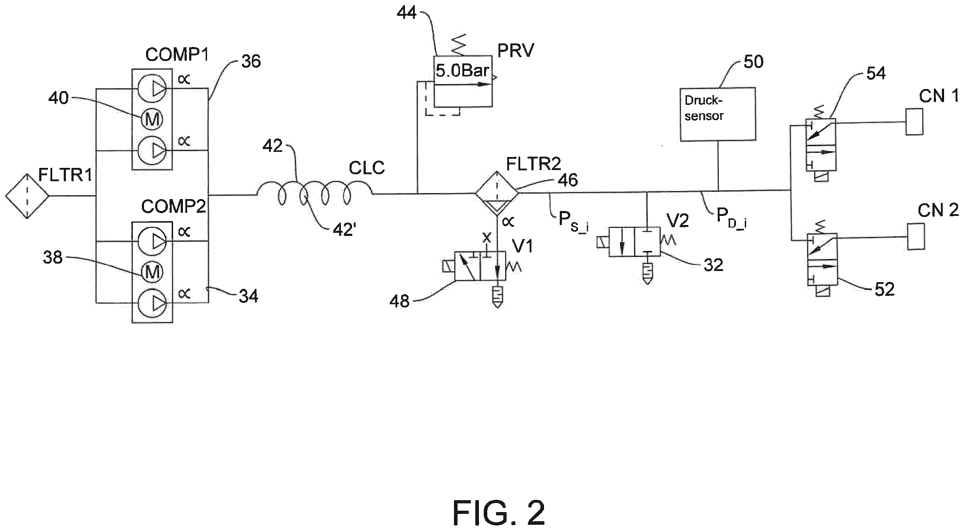

Figur 1 zeigt ein Druckwellengerät 10 zur Behandlung eines menschlichen oder tierischen Körpers (nicht dargestellt) mit einem pneumatischen Antrieb 14 zur Erzeugung einer Druckwelle 16 zur Einkopplung in den menschlichen oder tierischen Körper. Das Druckwellengerät 10, insbesondere der pneumatische Antrieb 14, weist mindestens einen Kompressor 18 zur Erzeugung von Quellengas bei einem Quellengasdruck P_{s_i} auf, wobei der Kompressor 18 einen Kompressormotor 20 umfasst. Es ist denkbar, dass statt einem Kompressor 18 mehrere Kompressoren 34, 36 (Fig. 2) von dem Druckwellengerät 10 umfasst sind. Ferner weist das Druckwellengerät 10 ein Handstück 12 auf, in welches das Quellengas über eine Verbindungsleitung 22 in einen Einlass 24 des Handstücks 12 einleitbar ist. Über das Handstück 12 ist die Druckwelle 16 in den menschlichen oder tierischen Körper einkoppelbar. Das Handstück 12 weist ein länglich ausgebildetes Führungsrohr 26 auf, in und entlang welchem ein Projektil 28 durch das Quellengas bis zu einem Prallkörper 30 beschleunigt werden kann. Der Prallkörper 30 dient als ein Übertragungselement zum Übertragen der Druckwelle 16 von dem Handstück 12 in den menschlichen oder tierischen Körper. Die Druckwelle 16 wird durch die beschleunigte Bewegung des Projektils 28 in dem Führungsrohr 26 erzeugt. Das Führungsrohr 26 dient folglich als Beschleunigungsstrecke für das Projektil 28. Die mittels des Druckwellengerätes 10 erzeugten Druckwellen 16 können bis zu 50 mm, in den menschlichen oder tierischen Körper eindringen.

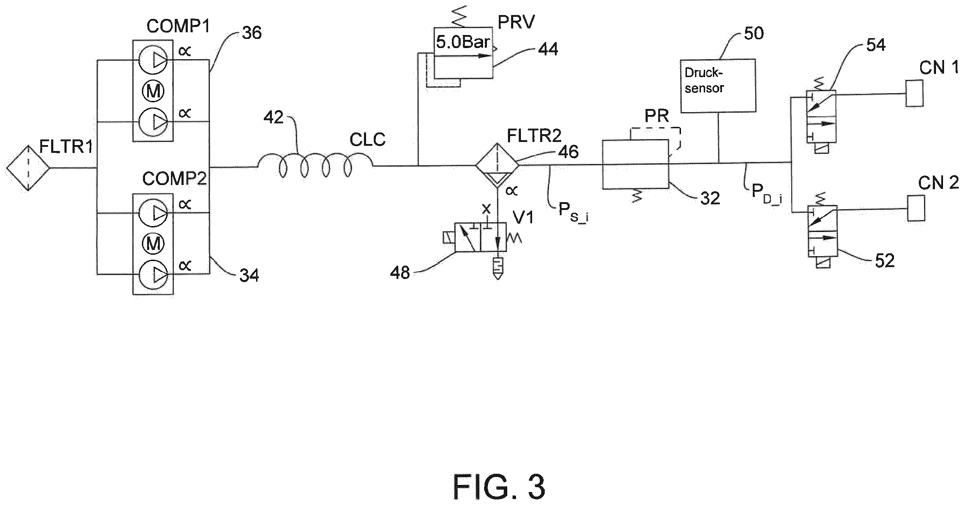

In Figur 1 ist in Zusammenschau mit den Figuren 2 und 3 erkennbar, dass vor bzw. an dem Einlass 24 des Handstücks 12 oder wahlweise am Antrieb 14 eine Druckregulierungsvorrichtung 32 zum Anpassen des Quellengasdrucks P_{s_i}, insbesondere zum Bereitstellen eines im Wesentlichen konstanten Druckwellenerzeugungsdrucks P_{D_i}, in dem Handstück 12 angeordnet ist. Mittels eines Reglers 19 ist der am Eingang vorbestimmte Druckwellenerzeugungsdruck bzw. der gewünschte Druckwellenerzeugungsdruck und mittels einer Aktivierungstaste 21 ist die Frequenz des Einleitens von Quellengas, das das Projektil beschleunigt, einstellbar. Mittels des Kompressors bzw. einem Kompressorsystem wird ein bestimmtes Leistungsniveau Lᵢ angesteuert, wwodurch ein erster Bereich von Druckwellenerzeugungsdrücken P_{D_i} durch den Regler 19 und ein erster Bereich von Frequenzen fᵢ durch die Aktivierungstaste 21 am Handstück 12 eingestellt werden kann. Hierbei bezeichnet der Quellengasdruck P_{s_i} einen Gasdruck, welcher von dem Kompressor 18 oder den mehreren Kompressoren 34, 36 erzeugt wird und vor einem Durchströmen der Druckregulierungsvorrichtung 32 vorherrscht. Ein Druckwellenerzeugungsdruck P_{D_i} resultiert hingegen aus dem Quellengasdruck P_{s_i} nach einem Durchströmen durch die Druckregulierungsvorrichtung 32.

Die Figuren 2 und 3 zeigen jeweils eine Alternative eines Schaltbildes des Druckwellengeräts 10 gemäß Figur 1. Gemäß Figur 2 umfasst der pneumatische Antrieb 14 des Druckwellengeräts 10 statt eines Kompressors 18 mit einem Kompressormotor 20 zwei Kompressoren 34, 36. Jeder Kompressor 34, 36 ist mit einem Kompressormotor 38, 40 verbunden, welcher den jeweiligen Kompressor 34, 36 antreibt. Die Kompressoren 34, 36 dienen folglich zum Komprimieren des Quellengases, wobei das Quellengas nach seiner Komprimierung eine Kühlzone durchläuft, in welcher eine Kühlvorrichtung 42, insbesondere eine Kühlspule 42' angeordnet ist. In der Kühlvorrichtung 42, insbesondere in der Kühlspule 42', wird das komprimierte Quellengas abgekühlt, bevor es über die Verbindungsleitung 22 in das Handstück 12 geleitet wird.

Zwischen der Kühlvorrichtung 42 und dem Einlass 24 des Handstückes 12 ist ferner ein Überdruckventil 44 zum Ableiten eines Überdrucks des Quellengases angeordnet. Das Überdruckventil 44 ist dazu ausgebildet, einen Druck, welcher einen vorgegebenen Druck, insbesondere einen maximalen Quellengasdruck P_{s_i}, übersteigt, automatisch aus der Verbindungsleitung 22 oder noch bevor das Quellengas in die Verbindungsleitung 22 gelangt, abzulassen. Hierdurch kann vermieden werden, dass ein Überdruck in dem Handstück 12 entsteht. Das Überdruckventil 44 ist beispielsweise dazu ausgebildet, einen Druck größer als z.B. 5 oder 10 bar, insbesondere aus der Verbindungsleitung 22, automatisch abzulassen. Selbstverständlich ist es denkbar, das Überdruckventil 44 auf eine andere Schaltschwelle des Gasdruckes zu kalibrieren, bei der das Überdruckventil 44 ggf. Gasdruck abläßt. Bevorzugt ist das Überdruckventil dazu ausgebildet, einen Gasdruck bis zu 25 bar Stand zu halten.

Besonders bevorzugt ist in der Verbindungsleitung 22 eine Kühlfalle 46 zum Auffangen von kondensiertem Quellengas, also des Kondensats, angeordnet. Es ist denkbar, dass das Überdruckventil 44 zwischen Kühlvorrichtung 42 und Kühlfalle 46 angeordnet ist. Die Kühlfalle 46 kann ferner mit einem Ablass- oder Entlüftungsventil 48, wie einem Magnetventil, verbunden sein. Durch das Entlüftungsventil 48 kann insbesondere nach jedem Gebrauch des Druckwellengeräts 10 das Druckwellengerät 10 vom Kondensat befreit und entlüftet werden. Hierdurch wird sichergestellt, dass kein restliches Kondensat und Quellengas in dem Druckwellengerät 10 nach einer Behandlung verbleibt. Ferner ist erfindungsgemäß an oder vor dem Einlass 24 des Handstücks 12 eine Druckregulierungsvorrichtung 32, insbesondere ein Druckregler, angeordnet, welche den Quellengasdruck P_{s_i} in der Verbindungsleitung 22 auf einen vorgegebenen, d.h. gewünschten, Druckwert, d.h. einem Druckwellenerzeugungsdruck P_{D_i}, einstellt, d.h. regelt. Es ist ferner denkbar, dass die Druckregulierungsvorrichtung 32 in dem pneumatischen Antrieb 14 angeordnet ist und der Gasdruck reguliert wird, bevor dieser in die Verbindungsleitung 22 strömt.

Zur Überprüfung des über die Druckregulierungsvorrichtung 32 eingestellten Druckwellenerzeugungsdrucks P_{D_i} ist ferner ein Drucksensor 50, beispielsweise in der Verbindungsleitung 22 oder an einer anderen geeigneten Position, angeordnet. Der Drucksensor 50 ist dazu ausgebildet, den Druckwellenerzeugungsdruck P_{D_i}, welcher nach Passieren der Druckregulierungsvorrichtung 32 in der Verbindungsleitung 22 strömt, zu erfassen und einem Nutzer, z.B. an einer Anzeige, anzuzeigen.

Die Figuren 2 und 3 unterscheiden sich allein in der Ausgestaltung der Druckregulierungsvorrichtung 32. Gemäß Figur 2 ist die Druckregulierungsvorrichtung 32 als ein Ventil, insbesondere als ein Magnetventil ausgebildet, welches einem Druck von beispielsweise bis zu 7 bar, bevorzugt bis zu 10 bar, standhalten kann. Gemäß Figur 3 ist die Druckregulierungsvorrichtung 32 als ein Druckminderer ausgebildet, welcher beispielsweise einem Druck von bis zu 7 bar, bevorzugt bis zu 10 bar, standhalten kann. Ein Druckminderer ist im Wesentlichen ein beliebiges Druckminderungsventil, welches zum Einbau in ein Schlauch- oder Leitungssystem ausgebildet ist, das trotz unterschiedlicher Drücke auf der Eingangsseite (Eingangsdruck, vorliegend der Quellengasdruck P_{s_i}) dafür sorgt, dass auf der Ausgangsseite ein bestimmter Ausgangsdruck (vorliegend der Druckwellenerzeugungsdruck P_{D_i}) nicht überschritten wird. Insbesondere unterscheidet sich ein Ablassventil von einem Druckminderer dahingehend, dass dessen Aufgabe darin besteht einen Überdruck abzubauen. Dies geschieht beispielsweise, indem ein Ventil geöffnet wird und Gas oder Flüssigkeit z. B. in die Umgebung abgelassen wird. Dabei kann das Ventil aktiv gesteuert werden oder aber als selbstständige Einheit mittels eines Federmechanismus bei einem einzustellenden Druck selbstständig öffnen. Beim Druckminderer wird hingegen kein Medium entlassen. Durch den Federmechanismus im Druckminderer wird gerade immer so viel Öffnungsquerschnitt freigegeben, dass sich hinter dem Druckminderer ein vorab einstellbarer Druck einstellt.

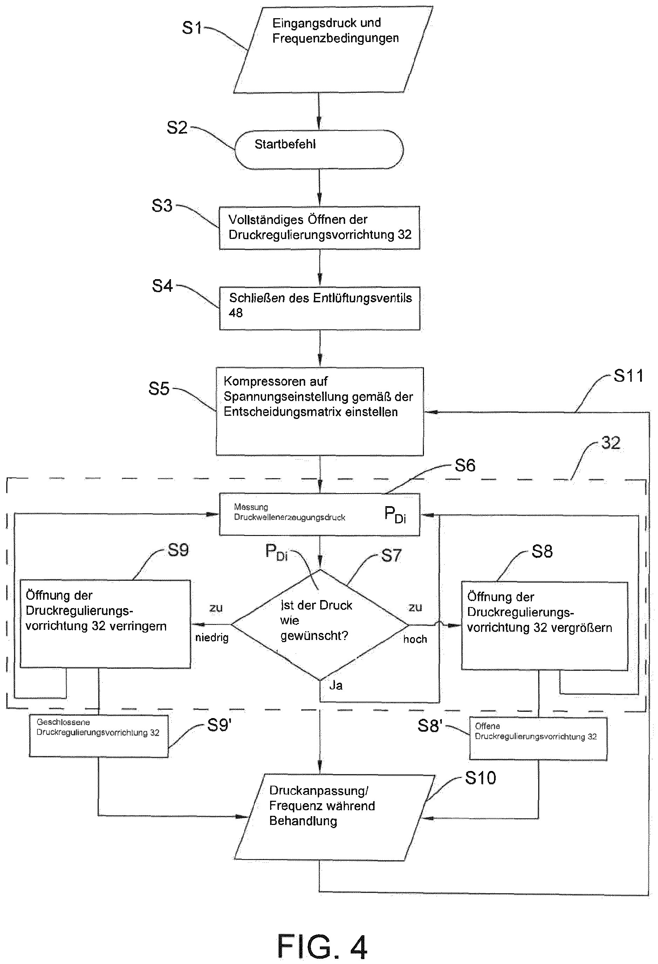

Figur 4 zeigt ein Ablaufschema eines bevorzugten Verfahrens zur Behandlung eines menschlichen oder tierischen Körpers mit Druckwellen 16 mit einem Druckwellengerät 10 wie hierin beschrieben. Das Verfahren umfasst die Schritte:
- Einschalten des mindestens einen Kompressors 18, 34, 36 zur Erzeugung von Quellengas,
- Durchleiten des Quellengases von dem mindestens einen Kompressor 18, 34, 36 zu dem Handstück 12, wobei
- der mindestens eine Kompressor 18, 34, 36 in einem Betriebszustand betrieben wird, der bei einem ersten Leistungsniveau einen ersten Quellengasdruck innerhalb eines Quellengasdruckbereich erzeugt,
- ein gewünschter Druckwellenerzeugungsdruck P_{D_i} durch Anpassung des ersten Quellengasdrucks erzeugt wird, indem eine Druckregulierungsvorrichtung 32 entsprechend geöffnet bzw. geschlossen wird.

Bei dem hierin vorgeschlagenen Verfahren können ferner, wie in Figur 4 in einem ersten Schritt S1 dargestellt, zunächst Eingaben zum anzusteuernden Leistungsniveau gemacht werden, d.h. es wird ein maximal gewünschter Druckwellenerzeugungsdruck und eine maximal gewünschte Frequenz eingegeben oder diese sind bereits zu dem entsprechenden Leistungsniveau im Druckwellengerät 10 hinterlegt. Insbesondere läßt sich für einen anzusteuernden Druckwellenerzeugungsdruckbereich bei einer bestimmten Frequenz (vorgegeben durch den Anwender) eine bestimmte Spannung, insbesondere eine von mehreren diskret auswählbaren Spannungen, für den Kompressor einstellen. Durch die Auswahl läßt sich insbesondere ein verschleißarmer und geräuscharmer Betriebszustand für den Kompressor auswählen. Nach Eingabe dieser Informationen kann in einem zweiten Schritt S2 das Verfahren als solches gestartet werden.

In einem dritten Schritt S3 wird zunächst die Druckregulierungsvorrichtung 32 geöffnet, so dass der erzeugte Quellengasdruck P_{s_i} aus dem oder den Kompressoren 34, 36 ungehindert durch die Verbindungsleitung 22 leitbar ist. In einem vierten Schritt S4 wird zeitgleich oder anschließend das Entlüftungsventil 48, welches in einem geöffneten Zustand zum Entlüften der Kühlfalle 46 dient, geschlossen. In einem fünften Schritt S5 kann anschließend der mindestens eine Kompressor 18, 34, 36 mit der vorgegebenen Spannung U angesteuert werden. Die vorgegebene Spannung kann in einer Steuerung hinterlegt sein. Die für einen verschleißarmen und/oder geräuscharmen Betrieb optimale Spannung U ist bevorzugt in Bezug auf eine vorgegebene diskrete Leistungsstufe Lᵢ hinterlegt. Nach Einschalten des mindestens einen Kompressors 18, 34, 36 strömt folglich der erzeugte Quellengasdruck über die Verbindungsleitung 22 zu dem Einlass 24 des Handstücks 12.

Beim Durchströmen der Verbindungsleitung wird das Quellengas durch die Druckregulierungsvorrichtung 32 von einem Quellengasdruck P_{s_i} auf den gewünschten Druckwellenerzeugungsdruck P_{D_i} eingeregelt. Beispielsweise kann ein Quellengasdruck P_{s_i} von 2 bar, welcher insbesondere einem ersten Leistungsniveau L₁ des mindestens einen Kompressors 18, 34, 36 zugeordnet ist, einem ersten Bereich 1 an Druckwellenerzeugungsdrücken P_{D_i} und einem ersten Bereich an Frequenzen fᵢ zugeordnet sein. Hierbei ist es nun denkbar, dass die Druckregulierungsvorrichtung 32 eine Minderung durchführt, um einen Druckwellenerzeugungsdruck P_{D_i} in einem Bereich zwischen 1 bar ≤ P_{D_i} ≤ 2 bar zu erreichen. Es ist ferner denkbar, dass neben einem ersten Bereich 1 noch ein zweiter und ein dritter Bereich 2, 3 vorgesehen sind, um zu einem zweiten oder dritten maximalen Quellengasdruck P_{s_i} einen gewünschten Druckwellenerzeugungsdruck P_{D_i} bei gewünschter Frequenz f einzustellen. Die Anzahl an Bereichen zum Einstellen des Druckwellenerzeugungsdrucks P_{D_i} ist vorliegend nicht auf drei Bereiche 1, 2, 3 beschränkt. Vielmehr können mehr oder weniger Bereiche vorgesehen sein. Die Anzahl der Bereiche ist insbesondere abhängig von der Anzahl an einstellbaren Leistungsniveaus des mindestens einen Kompressors.

Mittels eines Drucksensors 50, welcher beispielsweise vor oder an dem Einlass 24 angeordnet ist, wird der aktuelle Druckwellenerzeugungsdruck P_{D_i} nach einem Passieren der Druckregulierungsvorrichtung 32 in der Verbindungsleitung 22 erfasst. Wird in einem siebten Schritt S7 durch Bildung einer Differenz zwischen erfasstem Druckwellenerzeugungsdruck P_{D_i} und gewünschtem Druckwellenerzeugungsdruck P_{D_i} bestimmt, dass der erfasste Druckwellenerzeugungsdruck P_{D_i} einem gewünschten Druckwellenerzeugungsdruck P_{D_i} entspricht, so bleiben die Einstellungen an der Druckregulierungsvorrichtung 32 unverändert und der Druckwellenerzeugungsdruck P_{D_i} wird gemäß dem sechsten Schritt S6 in einem zeitlichen Abstand weiter erfasst. Wird in dem siebten Schritt S7 hingegen festgestellt, dass der gemessene Druckwellenerzeugungsdruck P_{D_i}, insbesondere in der Verbindungsleitung 22, den vorgegebenen Druckwellenerzeugungsdruck P_{D_i} überschreitet, d.h. zu hoch ist, so wird in einem achten Schritt S8 die Druckregulierungsvorrichtung 32 soweit geöffnet, dass ein erhöhter Druckwellenerzeugungsdruck P_{D_i}, insbesondere aus der Verbindungsleitung 22, entweichen kann (Ein Öffnen der Druckregulierungsvorrichtung 32 ist durch Schritt S8' wiedergegeben). Hierdurch kann vermieden werden, dass ein erhöhter Gasdruck, nämlich ein erhöhter Druckwellenerzeugungsdruck P_{D_i}, in das Handstück 12 eindringt und für die Behandlung unerwünscht ist.

Ferner wird gemäß dem sechsten Schritt S6 mittels des Drucksensors 50 der Druckwellenerzeugungsdruck P_{D_i} in der Verbindungsleitung 22 erfasst. Wird in dem siebten Differenzbildungs-Schritt S7 festgestellt, dass der gewünschte Druckwellenerzeugungsdruck unterhalb des erfassten Druckwellenerzeugungsdruck P_{D_i}, liegt, d.h. der vorbestimmte Druckwellenerzeugungsdruck P_{D_i} zu gering ist, so wird die Druckregulierungsvorrichtung 32 zumindest teilweise geschlossen (Ein Schließen der Druckregulierungsvorrichtung 32 ist durch Schritt S 9' wiedergegeben). Hierdurch wird erreicht, dass weniger Gas abgeblasen wird, so dass sich ein erhöhter Druckwellenerzeugungsdruck P_{D_i} in der Verbindungsleitung 22, insbesondere am oder vor dem Einlass 24 des Handstücks 12 einstellt.

Durch die Schritte S7 bis S9 wird erreicht, dass eine Druckanpassung während einer Behandlung automatisch erfolgt. Hierdurch wird folglich sichergestellt, dass die Druckwelle 16 wie vorgegeben bei einem gewünschten Druckwellenerzeugungsdruck P_{D_i} erzeugt werden kann. Ferner wird wiederum gemäß dem sechsten Schritt S6 mittels des Drucksensors 50 der Gasdruck, d.h. der Druckwellenerzeugungsdruck P_{D_i} und/oder der Quellengasdruck P_{s_i}, in der Verbindungsleitung 22 erfasst.

Falls es für die Behandlung mit Druckwellen notwendig ist, bzw. falls sich der gewünschte Druckwellenerzeugungsdruck P_{D_i} nicht (mehr) durch das Öffnen/Schlie-ßen in den Schritten S8', S9' einstellen läßt, kann in einem zehnten Schritt S10 das Leistungsniveau des oder der Kompressoren in eine andere Stufe "geschaltet" werden, d.h. die Drehzahl mit welcher der oder die Kompressoren für die Erzeugung von Druckwellen 16 betrieben werden, wird in eine höhere oder niedrigere Stufe geschaltet. Nach einer solchen Modifizierung gemäß Schritt S10 kann in einem elften Schritt S11 eine Anpassung des Druckwellenerzeugungsdrucks P_{D_i} gemäß der fünften bis zehnten Schritte S5 bis S10 erneut erfolgen.

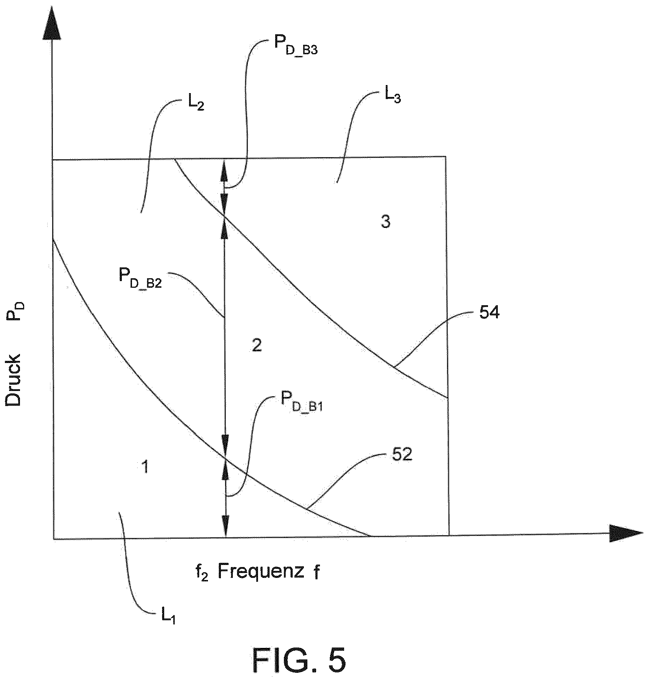

Figur 5 zeigt beispielshaft ein Betriebszustandsdiagramm. Für jedes Paar aus vorgesehener Frequenz f und vorgesehenem Druckwellenerzeugungsdruck P_{D} ist eine Spannung bzw. Betriebsspannung am Kompressor vorgesehen. Die Graphen in Figur 5 zeigen vorgegebene erste und zweite Schaltschwellen 52, 54 für die Leistungssniveaus 1, 2 und 3,, mit welchen eine Behandlung des menschlichen oder tierischen Körpers mit Druckwellen 16 durchgeführt werden soll. Die Bereiche 1, 2, 3 zwischen den Schaltschwellen 52, 54 sind den an dem mindestens einen Kompressor 18, 34, 36 einstellbaren diskreten Drehzahlen, d.h. Leistungsniveaus L_{1,2,3} zugeordnet. Wird beispielsweise ein zweites Leistungsniveau L₂ eingestellt, welches einem Bereich 2 zugeordnet ist, so ist in der Steuerung hinterlegt, dass ein bestimmter zweiter Bereich an Druckwellenerzeugungsdrücken P_{D_B2} bei einer bestimmten Frequenzen f₂ mittels der Druckregulierungsvorrichtung 32 eingeregelt werden kann.. Dies bedeutet, dass die Druckregulierungsvorrichtung 32 entweder zumindest teilweise geöffnet, oder teilweise geschlossen wird oder unverändert verbleibt. Wird beispielsweise eine dritte Leistungsstufe L₃ eingestellt, welche einem dritten Bereich 3 zugeordnet ist, so ist in der Steuerung hinterlegt, dass bei der bestimmten Frequenz f₂ ein bestimmter dritter Bereich an Druckwellenerzeugungsdrück P_{D_B3} oberhalb einer zweiten Schaltschwelle 54 ansteuerbar ist, d.h. die Druckregulierungsvorrichtung 32 kann dann zumindest teilweise geöffnet oder geschlossen werden, um den gewünschten Druckwellenerzeugungsdruck P_{D_i} innerhalb dieses dritten Druckbereichs P_{D_B3} einzustellen.

Wird beispielsweise eine erste Leistungsstufe L₁ eingestellt, welcher einem ersten Bereich 1 zugeordnet ist, so ist in der Steuerung hinterlegt, dass bei der bestimmten Frequenz f₂ ein bestimmter erster Bereich von Druckwellenerzeugungsdrücken P_{D_B1} ansteuerbar ist.

Mit anderen Worten: Je nach vorbestimmter Frequenz fⱼ lassen sich für jedes Leistungsniveau Lᵢ bestimmte Bereiche an regelbaren Druckwellenerzeugungsdrücken P_{D_Bi} einregeln und je nach vorbestimmtem Druckwellenerzeugungsdruck P_{D_j} lassen sich für jedes Leistungsniveau Lᵢ bestimmte Bereiche an regelbaren Frequenzen f_{Bi} einregeln.

In einer Steuerung (nicht gezeigt) sind zu einer vorgegebenen Schaltfrequenz f und einem vorgegebenen Druckwellenerzeugungsdruck P_{D_i} entsprechende Spannungseinstellungen U für den oder die Kompressoren 18, 34, 36 hinterlegt. Gemäß Figur 5 ergibt sich somit, dass bei einer festen Frequenz f der Druckwellenerzeugungsdruck P_{D_i} innerhalb der Schaltschwellen 52, 54 in einer Leistungsstufe verändert wird (man bewegt sich entlang einer Vertikalen) oder bei einem festen Druckwellenerzeugungsdruck P_{D_i} die Frequenz f verändert werden kann (man bewegt sich entlang einer Horizontalen). Abhängig von der Frequenz f und/oder von dem Druckwellenerzeugungsdruck P_{D_i} wird das Leistungsniveau L durch den Betrieb des oder der Kompressoren 18, 34, 36 angepasst. Selbstverständlich umfasst die vorgeschlagene Lösung auch eine Bewegung entlang des Graphen der Figur 5, welche sich aus einer horizontalen und vertikalen Bewegung zusammensetzt, d.h. eine Veränderung von Druckwellenerzeugungsdruck P_{D}und Frequenz f.

Es versteht sich, dass die wenigen Bereiche 1, 2, 3 und die Schaltschwellen 52, 54 der Figur 5 rein beispielhaft sind. Es ist denkbar, dass wesentlich mehr Bereiche und Schaltschwellen in der Steuerung hinterlegt sind, und somit ein Stufenmodul mit einer Mehrzahl an fest einstellbaren Stufen, d.h an diskreten gestuften Leistungsniveaus L hinterlegt ist. Ferner versteht sich von selbst, dass das Druckwellengerät 10 eine Mehrzahl an Kompressoren 18 aufweisen kann, wobei jeder oder viele Kompressoren 18 einen eigenen Kompressormotor 20 aufweisen kann/können. Die Verwendung mehrerer Kompressoren 18 vermindert die Geräuschkulisse.

### Bezugszeichenliste:

- 10: Druckwellengerät
- 12: Handstück
- 14: pneumatischer Antrieb
- 16: Druckwelle
- 18: Kompressor
- 19: Regler
- 20: Kompressormotor
- 21: Aktivierungstaste
- 22: Verbindungsleitung
- 24: Einlass
- 26: Führungsrohr
- 28: Projektil
- 30: Prallkörper
- 32: Druckregulierungsvorrichtung
- 34: Kompressor
- 36: Kompressor
- 38: Kompressormotor
- 40: Kompressormotor
- 42: Kühlvorrichtung
- 42': Kühlspule
- 44: Überdruckventil
- 46: Kühlfalle
- 48: Entlüftungsventil
- 50: Drucksensor
- 52: erste Schaltschwelle
- 54: zweite Schaltschwelle
- 1-3: Bereiche

- P_{D_i}: Druckwellenerzeugungsdruck
- P_{s_i}: Quellengasdruck
- Lᵢ: Lastbereich bzw. Leistungsniveau bzw. Leistungsstufe
- F: Frequenz
- P: Druck
- U: Spannung
- S1-S11: Schritte

## Patentansprüche

1. Druckwellengerät (10) zur Behandlung eines menschlichen oder tierischen Körpers mit einem pneumatischen Antrieb (14) zur Erzeugung einer Druckwelle (16) zur Einkopplung in den menschlichen oder tierischen Körper, mit mindestens einem Kompressor (18, 34, 36) zur Erzeugung von Quellengas und
mit einem Handstück (12), in welches das Quellengas einleitbar ist und mittels welchem eine Druckwelle (16) erzeugt wird, und
mit einer Druckregulierungsvorrichtung (32) zur Einstellung eines Druckwellenerzeugungsdrucks (P_{D}) zur Erzeugung der Druckwelle (16),
wobei
der mindestens eine Kompressor (18, 34, 36) zur Erzeugung von Quellengas stufenweise auf mindestens zwei Leistungsniveaus (Lᵢ) einstellbar ist, und die Druckregulierungsvorrichtung (32) den Druckwellenerzeugungsdruck (P_{D_i}) durch Anpassung des Quellengasdrucks (P_{s_i}) auf jedem Leistungsniveau Lᵢ regelt, **dadurch gekennzeichnet, dass** die Druckregulierungsvorrichtung (32) mindestens einen Drucksensor (50) zum Bestimmen eines Druckwellenerzeugungsdrucks P_{D_i} aufweist.

2. Druckwellengerät (10) nach Anspruch 1,
**dadurch gekennzeichnet, dass**
jedes Leistungsniveau (Lᵢ) durch einen Bereich an Druckwellenerzeugungsdruckwerten (P_{D_Bi}) und Frequenzen (f_{Bi}) der Aktivierung der Druckwellen (16) bestimmt ist.

3. Druckwellengerät (10) nach einem der Ansprüche 1 oder 2,
**dadurch gekennzeichnet, dass**
die Auswahl eines Leistungsniveaus (Lᵢ) anhand einer Tabelle erfolgt, in der die jeweiligen Bereiche an Druckwellenerzeugungsdruckwerten (P_{D_Bi}) und Frequenzen (f_{Bi}) hinterlegt sind.

4. Druckwellengerät (10) nach einem der vorhergehenden Ansprüche,
**dadurch gekennzeichnet, dass**
der mindestens eine Kompressor (18, 34, 36) von einem ersten Betriebszustand mit einem ersten Leistungsniveau (L₁) in einen zweiten Betriebszustand mit einem zweiten Leistungsniveaus (L₂) schaltbar ist, um durch die Druckregulierungsvorrichtung (32) einen zweiten Druckwellenerzeugungsdruck (P_{D_2}) einzustellen, sobald der im ersten Betriebszustand erzeugte Druckwellenerzeugungsdruck (P_{D_1}) bei einer bestimmten Frequenz (fᵢ) nicht mehr erreicht wird.

5. Druckwellengerät (10) nach einem der vorhergehenden Ansprüche,
**dadurch gekennzeichnet, dass**
die Druckregulierungsvorrichtung (32) einen Druckminderer oder ein Ablassventil aufweist, das insbesondere ein Ventil wie beispielsweise ein Magnetventil, aufweist, wobei der Druckminderer und/oder das Ablassventil ein Proportionalventil umfasst.

6. Druckwellengerät (10) nach einem der vorhergehenden Ansprüche,
**dadurch gekennzeichnet, dass**
mindestens zwei Kompressoren (34, 36) mit einem oder mit je einem Motor (38, 40) zur Ansteuerung der Kompressoren (24, 36) verbunden ist/sind.

7. Druckwellengerät (10) nach einem der vorhergehenden Ansprüche,
**dadurch gekennzeichnet, dass**
zwischen dem mindestens einen Kompressor (18, 34, 36) und der Druckregulierungsvorrichtung (32) eine Kühlfalle (46) zum Auffangen von abgekühltem, kondensiertem Quellengas angeordnet ist.

8. Druckwellengerät (10) nach einem der vorhergehenden Ansprüche,
**dadurch gekennzeichnet, dass**
zwischen dem mindestens einen Kompressor (18, 34, 36) und der Druckregulierungsvorrichtung (32) ein Überdruckventil (44) bzw. Ablassventil zum Ableiten eines Überdrucks des Quellengases vorgesehen ist.

9. Druckwellengerät (10) nach einem der vorhergehenden Ansprüche,
**dadurch gekennzeichnet, dass**
das Druckwellengerät (10) mittels eines ansteuerbaren Entlüftungsventils (48), insbesondere eines Magnetventils, entlüftbar ist.

## Claims

1. A pressure wave device (10) for treating a human or animal body, comprising a pneumatic drive (14) for generating a pressure wave (16) for coupling into the human or animal body, comprising
at least one compressor (18, 34, 36) for generating source gas, and
a handpiece (12) into which the source gas can be introduced and by means of which a pressure wave (16) is generated, and
a pressure regulation device (32) for setting a pressure wave generation pressure (PD) for generating the pressure wave (16),
wherein said at least one compressor (18, 34, 36) is stepwise adjustable to at least two performance levels (Lᵢ) for generating source gas, and
the pressure regulation device (32) regulates the pressure wave generation pressure (P_{D_i}) by adjusting the source gas pressure (P_{s_i}) at each performance level Lᵢ, **characterized in that** the pressure regulation device (32) comprises at least one pressure sensor (50) for determining a pressure wave generation pressure P_{D_i}.

2. The pressure wave device (10) according to claim 1,
**characterized in that**
each performance level (Lᵢ) is determined by a range of pressure wave generation pressure values (P_{D_Bi}) and frequencies (f_{Bi}) of activation of the pressure waves (16).

3. The pressure wave device (10) according to one of claims 1 or 2,
**characterized in that**
a performance level (Lᵢ) is selected on the basis of a table in which the respective ranges of pressure wave generation pressure values (P_{D_Bi}) and frequencies (f_{Bi}) are stored.

4. The pressure wave device (10) according to any one of the preceding claims,
**characterized in that**
the at least one compressor (18, 34, 36) can be switched from a first operating state with a first performance level (L₁) to a second operating state with a second performance level (L₂) in order to set a second pressure wave generation pressure (P_{D_2}) by the pressure regulation device (32) as soon as the pressure wave generation pressure (P_{D_1}) generated in the first operational state is no longer reached at a specific frequency (fᵢ).

5. The pressure wave device (10) according to any one of the preceding claims,
**characterized in that**
the pressure regulation device (32) comprises a pressure reducer or a drain valve comprising in particular a valve such as a solenoid valve, wherein the pressure reducer and/or the drain valve comprises a proportional valve.

6. The pressure wave device (10) according to any one of the preceding claims,
**characterized in that**
at least two compressors (34, 36) are connected to one or respectively one motor (38, 40) for driving the compressors (24, 36).

7. The pressure wave device (10) according to any one of the preceding claims,
**characterized in that**
a cold trap (46) for collecting cooled, condensed source gas is arranged between the at least one compressor (18, 34, 36) and the pressure regulation device (32).

8. The pressure wave device (10) according to any one of the preceding claims,
**characterized in that**
a relief valve (44) or drain valve is provided between the at least one compressor (18, 34, 36) and the pressure regulation device (32) for discharging an excess pressure of the source gas.

9. The pressure wave device (10) according to any one of the preceding claims,
**characterized in that**
the pressure wave device (10) can be vented by means of a controllable venting valve (48), in particular a solenoid valve.

## Revendications

1. Appareil à ondes de pression (10) pour le traitement d'un corps humain ou animal, comprenant un entraînement pneumatique (14) pour générer une onde de pression (16) destinée à être injectée dans le corps humain ou animal,
comprenant au moins un compresseur (18, 34, 36) pour générer du gaz source, et
comprenant une pièce à main (12) dans laquelle le gaz source peut être introduit et au moyen de laquelle une onde de pression (16) est générée, et comprenant un dispositif de régulation de pression (32) pour régler une pression de génération d'onde de pression (P_{D}) pour générer l'onde de pression (16),
dans lequel
ledit au moins un compresseur (18, 34, 36) pour générer du gaz source est réglable par étapes à au moins deux niveaux de puissance (Lᵢ), et
le dispositif de régulation de pression (32) régule la pression de génération d'onde de pression (P_{D_i}) en ajustant la pression de gaz source (P_{s_i}) à chaque niveau de puissance Lᵢ,
**caractérisé en ce que**
le dispositif de régulation de pression (32) comprend au moins un capteur de pression (50) pour déterminer une pression de génération d'onde de pression P_{D_i}.

2. Appareil à ondes de pression (10) selon la revendication 1,
**caractérisé en ce que**
chaque niveau de puissance (Lᵢ) est déterminé par une plage de valeurs de pression de génération d'onde de pression (P_{D_Bi}) et de fréquences (f_{Bi}) d'activation des ondes de pression (16).

3. Appareil à ondes de pression (10) selon l'une des revendications 1 ou 2, **caractérisé en ce que**
la sélection d'un niveau de puissance (Lᵢ) est effectuée à l'aide d'un tableau dans lequel sont stockées les plages respectives de valeurs de pression de génération d'onde de pression (P_{D_Bi}) et de fréquences (f_{Bi}).

4. Appareil à ondes de pression (10) selon l'une des revendications précédentes,
**caractérisé en ce que**
ledit au moins un compresseur (18, 34, 36) peut être commuté d'un premier état de fonctionnement avec un premier niveau de puissance (L₁) à un deuxième état de fonctionnement avec un deuxième niveau de puissance (L₂), afin de régler une deuxième pression de génération d'onde de pression (P_{D_2}) par le dispositif de régulation de pression (32), dès que la pression de génération d'onde de pression (P_{D_1}) générée dans le premier état de fonctionnement n'est plus atteinte à une fréquence déterminée (fᵢ).

5. Appareil à ondes de pression (10) selon l'une des revendications précédentes,
**caractérisé en ce que**
le dispositif de régulation de pression (32) comprend un réducteur de pression ou une vanne de décharge comprenant en particulier une vanne telle qu'une électrovanne, le réducteur de pression et/ou la vanne de décharge comprenant une vanne proportionnelle.

6. Appareil à ondes de pression (10) selon l'une des revendications précédentes,
**caractérisé en ce que**
au moins deux compresseurs (34, 36) sont reliés à un moteur ou à un moteur respectif (38, 40) destiné à piloter les compresseurs (24, 36).

7. Appareil à ondes de pression (10) selon l'une des revendications précédentes,
**caractérisé en ce que**
un piège de refroidissement (46) destiné à recueillir le gaz source refroidi et condensé est disposé entre ledit au moins un compresseur (18, 34, 36) et le dispositif de régulation de pression (32).

8. Appareil à ondes de pression (10) selon l'une des revendications précédentes,
**caractérisé en ce que**
une vanne de surpression (44) ou une vanne de décharge destinée à évacuer une surpression du gaz source est prévue entre ledit au moins un compresseur (18, 34, 36) et le dispositif de régulation de pression (32).

9. Appareil à ondes de pression (10) selon l'une des revendications précédentes,
**caractérisé en ce que**
l'appareil à ondes de pression (10) peut être purgé au moyen d'une vanne de purge pilotable (48), en particulier au moyen d'une électrovanne.
